# FASCICULE DE BREVET EUROPEEN

(11) **EP 0 615 732 B1**
(45) Date de publication et mention de la délivrance du brevet: **26.09.2001**
(21) Numéro de dépôt: 94420047.6
(22) Date de dépôt: 11.02.1994
(51) Int. Cl.: A61F 2/08, A61B 17/58

(54) **Vis d'interférence biorésorbable**
Bioabsorbierende Interferenzschraube
Bioabsorbable interference screw

(30) Priorité: 12.02.1993 FR 9301871
(43) Date de publication de la demande: 21.09.1994
(73) Titulaire: PHUSIS, 38420 Le Versoud (FR)
(72) Inventeur: Chambat, Pierre, F-69110 Saint Foy les Lyon (FR); Fayard, Jean-Philippe, F-42170 Saint Just Saint Rambert (FR)
(74) Mandataire: Derambure, Christian

(56) Documents cités:
- EP-A- 0 451 932
- EP-A- 0 502 698
- WO-A-89/09030
- US-A- 4 950 270

## Description

La présente invention est relative aux vis d'interférence réalisées en matière biorésorbable.

Les vis de ce genre, mais réalisées en matière métallique usuelle (titane, inox, etc...), sont utilisées en chirurgie pour des ligamento-plasties et en particulier pour celles du ligament croisé antérieur du genou. Lorsque ce ligament est lésé, il faut le remplacer en utilisant un transplant, provenant généralement du tendon rotulien et qui comporte le tendon proprement dit, et à ses deux extrémités une petite barrette osseuse. Le transplant est passé dans deux tunnels, l'un fémoral, l'autre tibial débouchant sur l'articulation du genou et à proximité des insertions du ligament croisé antérieur. Le transplant vient remplacer ce ligament, ses deux barrettes osseuses extrêmes devant venir se positionner au débouché des tunnels tibial et fémoral.

On utilise des vis d'interférence pour verrouiller le transplant en les engageant dans le même axe que le tunnel, de manière qu'elles coincent chaque barrette osseuse par rapport à la paroi du tunnel à la manière d'un coin. Les vis d'interférence métalliques utilisées jusqu'à présent posent plusieurs problèmes :
- la position de ces vis complètement enfouies dans une structure osseuse rend leur extraction difficile et délicate.
- la présence desdites vis se révèle gênante en cas de nouvelle intervention chirurgicale dans la zone concernée, par exemple ostéotomie, pose d'une prothèse, etc...
- les filets tranchants des vis peuvent détériorer à la pose ou à long terme la structure fibreuse du transplant au niveau de ses insertions sur les barrettes osseuses.
- à long terme, la présence des vis métalliques peut altérer les structures osseuses proximales.
- enfin, un phénomène d'intolérance des implants métalliques à long terme ne peut pas être écarté.

On connaît du document EP-A-502 698 une vis d'interférence biorésorbable sans tête réalisée à partir d'un homopolymère d'acide Lactique à haute pureté chimique.

Un des problèmes qui se pose avec ce type de vis est que, lors de sa fabrication par moulage par injection, la viscosité intrinsèque de l'homopolymère diminue fortement ce qui ne permet pas de garantir l'obtention d'une vis présentant des propriétés mécaniques optimales.

L'invention vise donc à remédier à ces inconvénients en proposant une vis d'interférence biorésorbable qui présente les caractéristiques physico-chimiques définies dans la revendication 1 de sorte à présenter une aptitude au moulage par injection améliorée.

Suivant l'invention, de telle vis d'interférence biorésorbables sont réalisées sous la forme d'une vis sans tête à filet rond fabriquée à partir d'un stéréocopolymère de l'acide lactique de haute pureté chimique, cette vis comportant un alésage axial traversant dont la majeure partie est constituée par un prisme creux, tandis que son extrémité avant est réalisée sous la forme d'un tronc de cône sur lequel s'étend le filet rond.

Le dessin annexé, donné à titre d'exemple, permettra de mieux comprendre l'invention, les caractéristiques qu'elle présente et les avantages qu'elle est susceptible de procurer :
Fig. 1 est une vue en perspective d'une vis d'interférence suivant l'invention associée à sa clé de serrage.
Fig. 2 est une coupe longitudinale de la vis suivant l'invention.
Fig. 3 en est une vue en bout suivant la flèche III de fig. 2.

On sait qu'une matière biorésorbable peut être constituée par un stéréocopolymère de l'acide lactique de haute pureté optique (copolymère des acides L et D-Lactiques).

On peut également utiliser des copolymères biorésorbables de l'acide lactique et d'un comonomère compatible, par exemple dérivé d'α-hydroxy-acides.

D'une façon générale, les copolymères utilisés pour réaliser des pièces biorésorbables contiennent au moins 90% de motifs dérivés de l'acide L-lactique afin d'assurer le maintien de propriétés mécaniques suffisantes pendant un temps approprié après mise en place des pièces en question.

Dans notre exemple, il sera préférable d'utiliser un stéréocopolymère d'acide lactique de haute pureté chimique, comportent au moins 95% de motifs dérivés de l'acide L-lactique.

Les vis d'interférence établies conformément à l'invention sont bien entendu fabriquées à partir des matières ci-dessus citées, mais on choisit dans la gamme un stéréo copolymère d'acide lactique de très grande pureté chimique, ayant une masse moléculaire supérieure à 250 000 et une très faible polydispersité, inférieure à 2. Il est impératif de respecter ces caractéristiques pour pouvoir réaliser les vis suivant l'invention par moulage par injection.

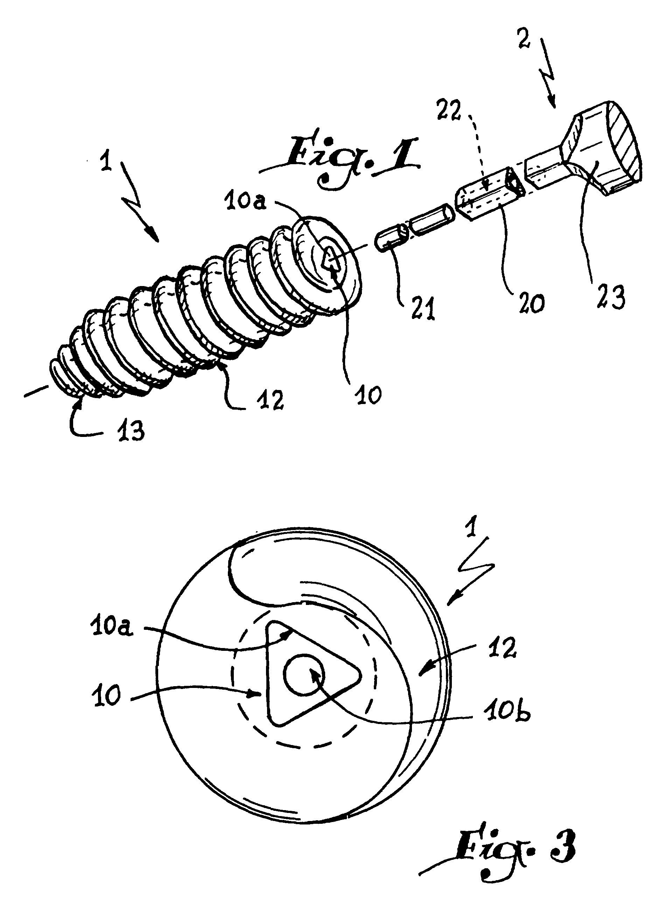

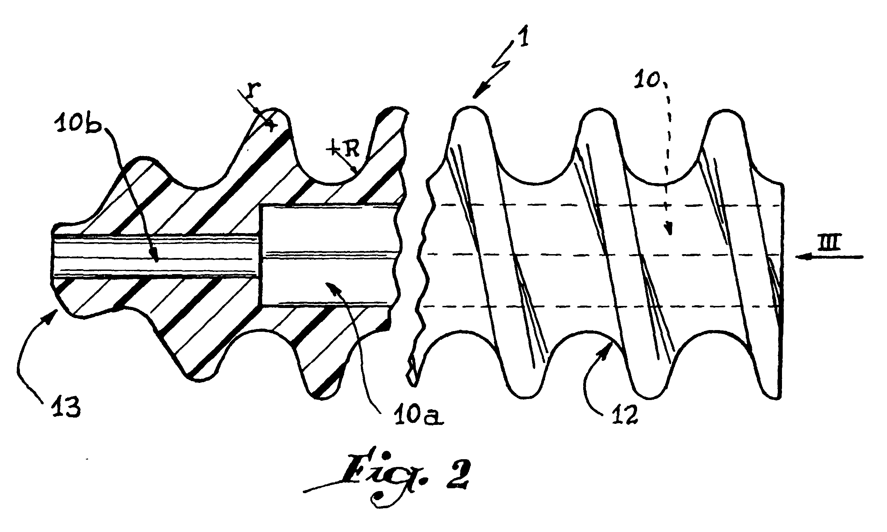

Les vis suivant l'invention sont des vis 1 sans tête à filet arrondi profond et comportant suivant leur axe géométrique un alésage 10 traversant. Comme illustré en fig. 2, cet alésage comporte sur la majeure partie de la longueur de la vis la forme d'un prisme 10a, tandis que sur la partie restante ledit alésage est un trou 10b à section transversale circulaire inférieure à celle du prisme creux 10a. Le prisme creux peut par exemple s'étendre sur les 4/5 de la longueur de la vis pour des raisons qu'on expliquera plus loin.

Le filet 12 de la vis 1 est du type dit rond, c'est-à-dire qu'il présente des fonds courbes de rayons R raccordés aux sommets arrondis suivant un rayon r par deux flancs déterminant entre eux un angle d'environ 30°. La profondeur du filet est assez importante et le rayon R est légèrement supérieur à celui r. Pour fixer les idées, si l'on envisage une vis de diamètre extérieur 9 mm, la profondeur du filet est de l'ordre de 1,7 mm. Bien entendu, la forme arrondie du filet évite que son sommet soit tranchant.

On observe aussi en fig. 2 que l'extrémité 13 de la vis, dont la longueur correspond en gros à celle de la partie cylindrique 10b, est tronconique suivant un angle d'environ 30° et le filet se poursuit sur cette partie tronconique pour faciliter sa mise en place dans l'os spongieux sans taraudage préalable.

La mise en place s'effectue au moyen d'un tournevis 2 (fig. 1) comportant une partie prismatique 20 de section égale au jeu près à celle du prisme creux 10a. Une broche 21 de diamètre inférieur à celui de la partie 10b de l'alésage 10 de la vis 1 est engagée à coulissement juste dans un alésage central borgne 22 du tournevis 2. Ainsi, une fois la partie 20 du tournevis engagée à fond dans le prisme creux 10a, la broche 21 présente une Longueur telle qu'elle dépasse à l'extérieur de la vis 1 au-delà de son extrémité tronconique, de manière à constituer un centrage dans l'os devant recevoir la vis. Bien entendu, le tournevis est muni d'un manche 23.

Comme indiqué plus haut, le prisme creux 10a de la vis est profond pour permettre une coopération étroite avec les facettes de la partie prismatique 20 du tournevis et assurer ainsi un couple de vissage réparti sur la majeure partie de la longueur de la vis, ce qui permet la mise en place de cette dernière sans taraudage préalable. Egalement le prisme creux 10a est inscrit dans un cercle dont le diamètre représente 70% à 80% du diamètre de l'âme de la vis pour une transmission optimale du couple de vissage. Le prisme creux 10a pourrait bien entendu présenter une autre section, mais la forme triangulaire choisie permet de conserver une quantité de matière plastique suffisante autour de ce prisme pour assurer la transmission du couple de vissage sans altérer la géométrie dudit prisme.

Il va de soi qu'on peut réaliser des vis d'interférence biorésorbables suivant la structure décrite précédemment dans des dimensions diverses en diamètre et en longueur sélectionnées comme étant les mieux adaptées à l'application envisagée.

Son procédé de fabrication est avantageusement un moulage par injection d'un stéréo-copolymère d'acide lactique tel que décrit ci-dessus.

On a ainsi réalisé une vis d'interférence biorésorbable dont la cinétique de résorption est lente et dont les propriétés mécaniques sont maintenues pendant un temps suffisant. Ainsi, l'hydrolyse de la matière est très progressive. On assure donc une bonne stabilité in-vivo, les produits qui sont relargués très progressivement au cours de la résorption sont métabolisés et éliminés par la voie naturelle . Pour fixer les idées, l'altération de la matière est infime pendant les trois premiers mois, la disparition complète de la vis n'intervenant qu'au délà de seize mois.

## Revendications

1. Vis d'interférence biorésorbable sans tête, du genre comprenant un alésage axial traversant (10) dont la majeure partie (10a) est réalisée sous la forme d'un prisme creux, la partie restante constituant un trou cylindrique (10b), réalisée à partir d'un stéréocopolymère d'acide lactique de haute pureté chimique, **caractérisé en ce qu'**il comporte au moins 90% de motifs dérivés de l'acide L-lactique, une masse supérieure à 250 000 et un indice de polydispersité inférieur à 2.

2. Vis d'interférence suivant La revendication 1, **caractérisée en ce qu'**elle comprend un filet (12) à profil arrondi avec un angle des flancs de l'ordre de 30° et un rayon de courbure au sommet (r) inférieur à celui des fonds de courbe (R) pour assurer une bonne stabilité mécanique.

3. Vis d'interférence suivant la revendication 1, **caractérisée en ce que** le prisme creux (10a) s'inscrit dans un cercle dont le diamètre représente 70% à 80% du diamètre de l'âme de la vis (1) pour conserver une quantité de matière plastique suffisante autour dudit prisme afin d'assurer la transmission du couple de vissage sans altérer sa géométrie.

4. Vis d'interférence suivant la revendication 1, **caractérisée en ce que** sa matière constitutive contient au moins 95% de motifs dérivés de l'acide L-lactique.

5. Vis d'interférence suivant la revendication 1, **caractérisée en ce qu'**elle est moulée par injection.

## Patentansprüche

1. Biologisch resorbierbare, kopflose Interferenzschraube mit einer axial durchgehenden Bohrung (10), deren größter Teil (10a) in Form eines hohlen Primas hergestellt ist, wobei der restliche Teil ein zylindrisches Loch (lOb) bildet, hergestellt aus einem chemisch hochreinen Milchsäure-Stereokopolymer, **dadurch gekennzeichnet, dass** sie mindestens 90% aus der L-Milchsäure stammende Einheiten, eine Masse von über 250 000 und ein Polydispersionszahl von weniger als 2 aufweist.

2. Interferenzschraube nach Anspruch 1, **dadurch gekennzeichnet, dass** sie ein Gewinde (12) mit abgerundetem Profil mit einem Flankenwinkel von etwa 30° und einen kleineren Krümmungsradius am Gipfel (r) als derjenige der Kurvenböden (R) aufweist, um eine gute mechanische Stabilität zu gewährleisten.

3. Interferenzschraube nach Anspruch 1, **dadurch gekennzeichnet, dass** das hohle Prisma (10a) in einem Kreis liegt, dessen Durchmesser 70% bis 80% des Durchmessers des Schraubenkerns (1) ausmacht, um eine ausreichende Menge von Kunststoff um das besagte Prisma herum zu behalten, um die Übertragung des Schraubmoments zu gewährleisten, ohne ihre Geometrie zu verschlechtern.

4. Interferenzschraube nach Anspruch 1, **dadurch gekennzeichnet, dass** ihr Werkstoff mindestens 95% aus der L-Milchsäure stammende Einheiten beinhaltet.

5. Interferenzschraube nach Anspruch 1, **dadurch gekennzeichnet, dass** sie im Spritzgussverfahren hergestellt wird.

## Claims

1. A headless bioresorbable interference screw, of the type comprising a through axial bore (10), the major part (10a) of which is produced in the form of a hollow prism, the remaining part constituting a cylindrical hole (lOb), produced from a lactic acid stereocopolymer with high chemical purity, **characterised in that** it contains at least 90% units derived from L-lactic acid, a mass greater than 250,000 and a polydispersity index of less than 2.

2. An interference screw according to Claim 1, **characterised in that** it comprises a thread (12) with a rounded profile with a flank angle of around 30° and a radius of curvature at the top (r) less than that of the bottoms of the curve (R) in order to ensure good mechanical stability.

3. An interference screw according to Claim 1, **characterised in that** the hollow prism (10a) fits within a circle whose diameter represents 70% to 80% of the diameter of the core of the screw (1) in order to preserve a sufficient quantity of plastics material around the said prism so as to ensure transmission of the torque without impairing its geometry.

4. An interference screw according to Claim 1, **characterised in that** its constituent material contains at least 95% units derived from L-lactic acid.

5. An interference screw according to Claim 1, **characterised in that** it is injection moulded.
